# EUROPÄISCHE PATENTSCHRIFT

(11) **EP 2 101 646 B1**
(45) Veröffentlichungstag und Bekanntmachung des Hinweises auf die Patenterteilung: **16.03.2011**
(21) Anmeldenummer: 07857160.1
(22) Anmeldetag: 29.12.2007
(51) Int. Cl.: A61B 5/151

(54) **STECHGERÄT**
PRICKING DEVICE
APPAREIL PERFORANT

(30) Priorität: 13.01.2007 EP 07000658; 01.08.2007 EP 07015132
(43) Veröffentlichungstag der Anmeldung: 23.09.2009
(73) Patentinhaber: Roche Diagnostics GmbH, 68305 Mannheim (DE); F. Hoffmann-La Roche AG, 4070 Basel (CH)
(72) Erfinder: KONYA, Ahmet, 67165 Waldsee (DE)
(74) Vertreter: Twelmeier Mommer & Partner
(86) Internationale Anmeldenummer: PCT/EP2007/011469
(87) Internationale Veröffentlichungsnummer: WO 2008/083844

(56) Entgegenhaltungen:
- EP-A- 1 714 613
- WO-A-2005/107596
- DE-B1- 2 803 345
- US-A1- 2003 199 789

## Beschreibung

Die Erfindung betrifft ein Stechgerät zum Gewinnen einer Körperflüssigkeitsprobe mit einem Aufnahmefach zum Aufnehmen eines Lanzettenvorratsbands mit mehreren Lanzetten, die quer zur Längsrichtung des Lanzettenvorratsbandes angeordnet sind und eine Lanzettenspitze zum Erzeugen einer Einstichwunde aufweisen, einer Fördereinrichtung, um durch Transportieren eines in das Aufnahmefach eingesetzten Lanzettenvorratsbandes die Lanzetten des Lanzettenvorratsbands nacheinander in eine Einstichposition zu bringen, und einem Stechantrieb, um eine Lanzette, die sich in der Einstichposition befindet, in eine Einstichbewegung zu versetzten. Die Erfindung betrifft ferner ein Stechsystem und eine Bandkassette mit einem Lanzettenvorratsband für Stechgerät sowie ein Verfahren zum Trennen einer Lanzettenspitze von einem Lanzettenvorratsband.

Derartige Stechgeräte und dazugehörende Lanzettenvorratsbänder, die quer zur Längsrichtung des Lanzettenvorratsbandes angeordnete Lanzetten enthalten, sind beispielsweise aus der US 2003/0199906 A1 und der DE 2803345 B1 bekannt. Die in diesen Veröffentlichungen beschriebenen Lanzettenvorratsbänder umfassen einen flexiblen Träger, auf dem die Lanzetten angeordnet sind und einen die Lanzetten bedeckenden Sterilschutz. Der Sterilschutz der bekannten Lanzettenvorratsbänder wird von einer Folie gebildet, die abgezogen werden muss, bevor die Lanzetten für einen Einstich verwendet werden können. Zum Entfernen des Sterilschutzes ist bei bekannten Stechsystemen ein relativ hoher Geräteaufwand erforderlich.

Aufgabe der Erfindung ist es deshalb, einen Weg aufzuzeigen, wie bei einem Stechgerät der Eingangs genannten Art mit geringerem Aufwand Lanzetten eines Lanzettenvorratsbandes für einen Einstich bereit gestellt werden können.

Die Aufgabe wird erfindungsgemäß gelöst durch eine Biegeeinrichtung, um einen Abschnitt des Lanzettenvorratsbands in Längsrichtung des Lanzettenvorratsbandes zu biegen und dadurch eine Lanzettenspitze einer Lanzette für einen Einstich von dem Lanzettenvorratsband zu trennen.

Eine erfindungsgemäße Biegeeinrichtung kann an einem Stechgerät angebracht sein. Möglich ist es insbesondere auch, dass die Biegeeinrichtung an einer Bandkassette angebracht ist, die zum Gebrauch in ein Aufnahmefach eines Stechgeräts eingelegt wird. Eine Bandkassette und ein Stechgerät, in das die Bandkassette zum Gebrauch eingelegt wird, bilden ein Stechsystem.

Ein erfindungsgemäßes Stechgerät oder Stechsystem kann jedoch auch ohne auswechselbare Bandkassetten verwirklicht werden, indem ein Austausch des Lanzettenvorratsbandes nicht vorgesehen ist und nach Gebrauch aller Lanzetten des Lanzettenvorratsbandes das Stechgerät bzw. Stechsystem als Wegwerfartikel entsorgt wird.

Im Rahmen der Anmeldung wird unter einer Lanzettenspitze das vordere, zum Einstich bestimmte Ende einer Lanzette verstanden.

Wird ein Lanzettenvorratsband, das mehrere quer zur Längsrichtung des Lanzettenvorratsbandes angeordnete Lanzetten enthält, in Längsrichtung gefaltet oder gebogen, so behält eine Lanzette in dem gebogenen Abschnitt des Lanzettenvorratsbands ihre Orientierung weitgehend bei. Dies führt dazu, dass sich die Spitze der Lanzette von dem Vorratsband abhebt und durch eine Sterilschutz- oder Trägerschicht hindurch bricht. Auf diese Weise kann eine Lanzettenspitze einer Lanzette für einen Einstich von dem Lanzettenvorratsband getrennt werden, so dass die Lanzette für einen Einstich bereit steht. Es ist nicht erforderlich, dass eine Lanzette durch das Biegen des Lanzettenvorratsbandes vollständig von dem Lanzettenvorratsband getrennt wird. Es genügt, wenn durch das Biegen die Lanzettenspitze, die bei einem Einstich in die Haut eines an das Stechgerät angelegten Körperteils eindringt, von dem Lanzettenvorratsband getrennt wird. Durch das Biegen wird ein vorderer Lanzettenbereich, zu dem die Lanzettenspitze gehört, von dem Lanzettenvorratsband getrennt.

Ein erfindungsgemäßes Stechgerät und ein Lanzettenvorratsband mit mehreren Lanzetten, die quer zur Längsrichtung des Lanzettenvorratsbandes angeordnet sind, bilden ein Stechsystem. Ein weiterer Aspekt der Erfindung betrifft ein Verfahren zum Trennen einer Lanzettenspitze von einem Lanzettenvorratsband, wobei das Lanzettenvorratsband in Längsrichtung gebogen wird, so dass sich die Lanzettenspitze von dem Lanzettenvorratsband abhebt, wobei zum Biegen des Bandes eine in einem Stechgerät angeordneten Biegeeinrichtung verwendet wird. Bevorzugt wird zum Biegen des Bandes mittels der Biegeeinrichtung auf eine Längskante des Lanzettenvorratsbandes eingewirkt.

Das Lanzettenvorratsband eines erfindungsgemäßen Stechsystems kann beispielsweise ähnlich wie aus der US 2003/0199906 A1 oder der DE 2803345 B1 bekannte Lanzettenvorratsbänder ausgebildet sein und eine flexible Trägerschicht, auf der die Lanzetten angeordnet sind und einen die Lanzetten bedeckenden Sterilschutz umfassen. Der Sterilschutz kann beispielsweise als Folie, insbesondere aus Kunststoff oder Papier, oder als Silikonschicht ausgebildet sein. Eine weitere Möglichkeit für das Lanzettenvorratsband besteht beispielsweise darin, Lanzetten auf einem Trägerband, beispielsweise aus Papier, Kunststoff- oder/und Metallfolie, anzuordnen und das Trägerband zu falten, so dass es die Lanzettenspitzen bedeckt und auf diese Weise vor schädlichen Umwelteinflüssen schützt. Prinzipiell ist es auch möglich, die Lanzetten auf einem Trägerband anzuordnen, das aufgewickelt wird, so dass die Lanzetten in einer so gebildeten Lanzettenvorratsbandrolle zwischen der Oberseite und der Unterseite des Trägerbands vor schädlichen Umwelteinflüssen geschützt sind.

Weitere Einzelheiten und Vorteile der Erfindung werden anhand von Ausführungsbeispielen unter Bezugnahme auf die beigefügten Zeichnungen erläutert. Die dabei beschriebenen Merkmale können einzeln oder in Kombination zum Gegenstand von Ansprüchen gemacht werden. Gleiche und ein der entsprechende Teil sind mit übereinstimmenden Bezugszahlen gekennzeichnet. Es zeigen:
- Fig. 1: ein Ausführungsbeispiel eines erfindungsgemäßen Stechgeräts;
- Fig. 2: eine schematische Innenansicht des in Figur 1 dargestellten Ausführungs- beispiels mit einem eingelegten Lanzettenvorratsband vor einem Biegevor- gang;
- Fig. 3: eine um 90° gedrehte Ansicht zur Figur 2;
- Fig.4: eine Darstellung gemäß Figur 2 nach dem Biegen des Lanzetten- vorratsbandes;
- Fig. 5: eine um 90° gedrehte Ansicht zu Figur 4;
- Fig. 6: die Position des Lanzettenvorratsbandes hinsichtlich der Biegeeinrichtung nach einem Einstich;
- Fig. 7: eine um 90° gedrehte Ansicht zu Figur 6;
- Fig. 8: das Lanzettenvorratsband in einer Probenaufnahmeposition;
- Fig. 9: eine um 90° gedrehte Ansicht zu Figur 8;
- Fig. 10: ein Ausführungsbeispiel einer Bandkassette in einer schematischen Darstel- lung;
- Fig. 11: das in Figur 10 dargestellte Ausführungsbeispiel in einer Seitenansicht;
- Fig. 12: eine schematische Darstellung eines weiteren Ausführungsbeispiels eines erfindungsgemäßen Stechgeräts in einer Draufsicht;
- Fig. 13: eine Seitenansicht zu Figur 12 vor einer Stichbewegung
- Fig. 14: eine Seitenansicht zu Figur 12 während einer Stichbewegung;
- Fig. 15: eine schematische Darstellung eines weiteren Ausführungsbeispiels eines erfindungsgemäßen Stechgeräts in einer Draufsicht vor einer Stichbewe- gung;.
- Fig. 16: eine Seitenansicht zu Figur 15 vor einer Stichbewegung;
- Fig. 17: eine Draufsicht des in Figur 15 gezeigten Ausführungsbeispiels während ei- ner Stichbewegung; und
- Fig. 18: eine Seitenansicht zu Figur 17 während einer Stichbewegung.

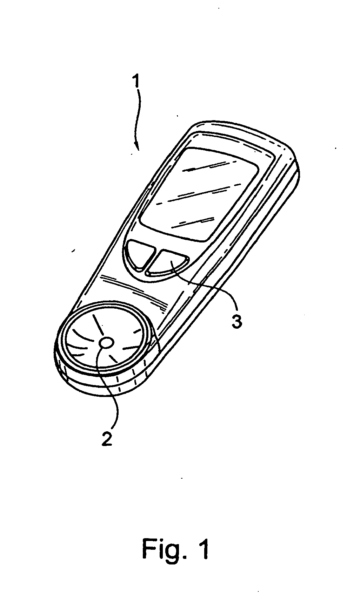

Figur 1 zeigt in einer schematischen Darstellung ein Ausführungsbeispiel eines erfindungsgemäßen Stechgeräts 1 zum Gewinnen einer Körperflüssigkeitsprobe. Das Stechgerät 1 weist eine Geräteöffnung 2 auf, gegen die ein Körperteil zum Erzeugen einer Einstichwunde gepresst wird. Das Stechgerät 1 umfasst ferner Bedienungselemente 3 in Form von Tasten und eine Anzeigeeinrichtung 4 in Form einer Flüssigkristallanzeige zum Anzeigen von Untersuchungsergebnissen. Das Stechgerät 1 hat ein Aufnahmefach (nicht dargestellt) für ein Lanzettenvorratsband. Das Aufnahmefach hat eine verschließbare Öffnung, die sich auf der Rückseite des in Figur 1 dargestellten Ausführungsbeispiels befindet.

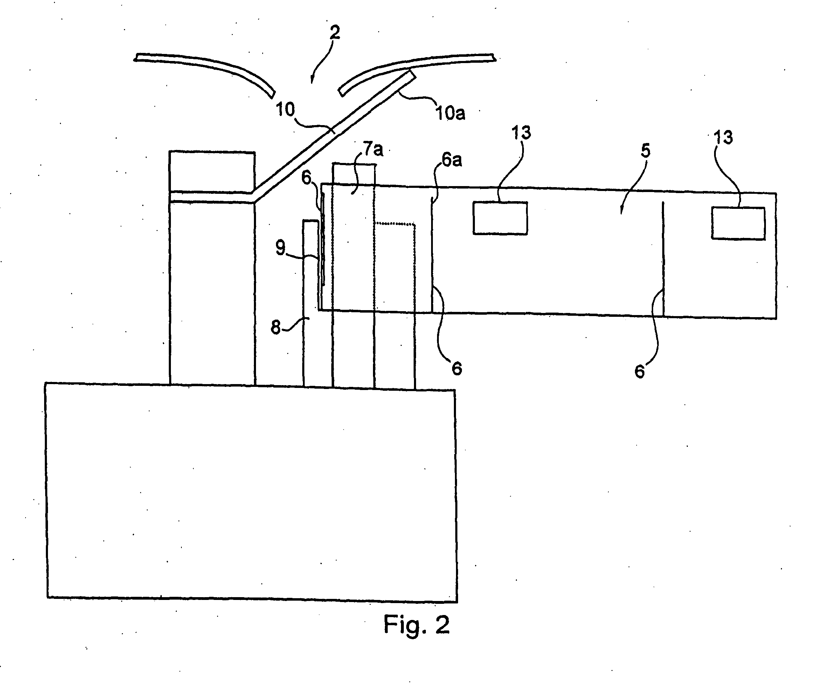

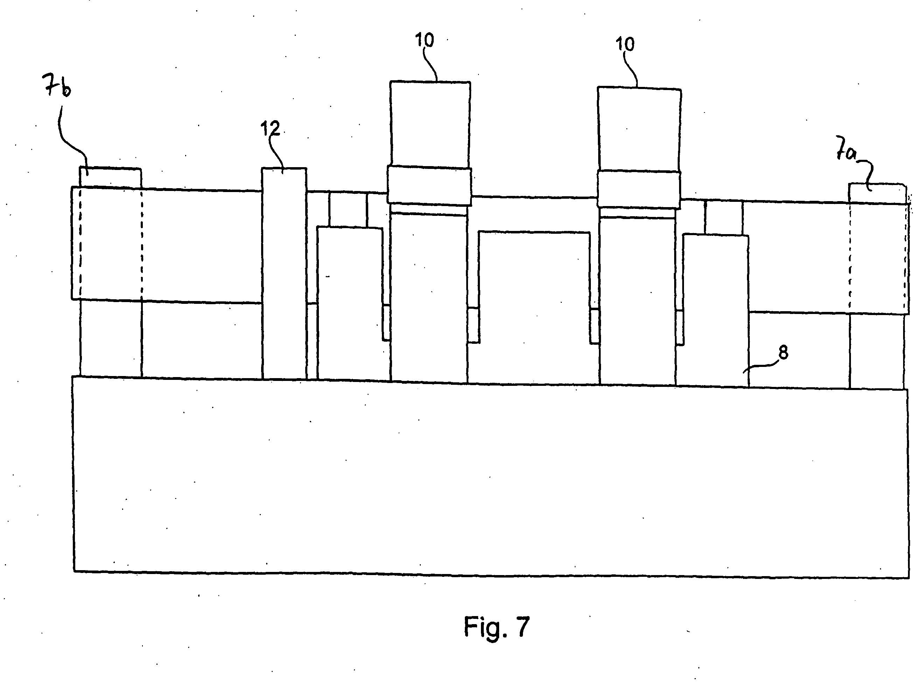

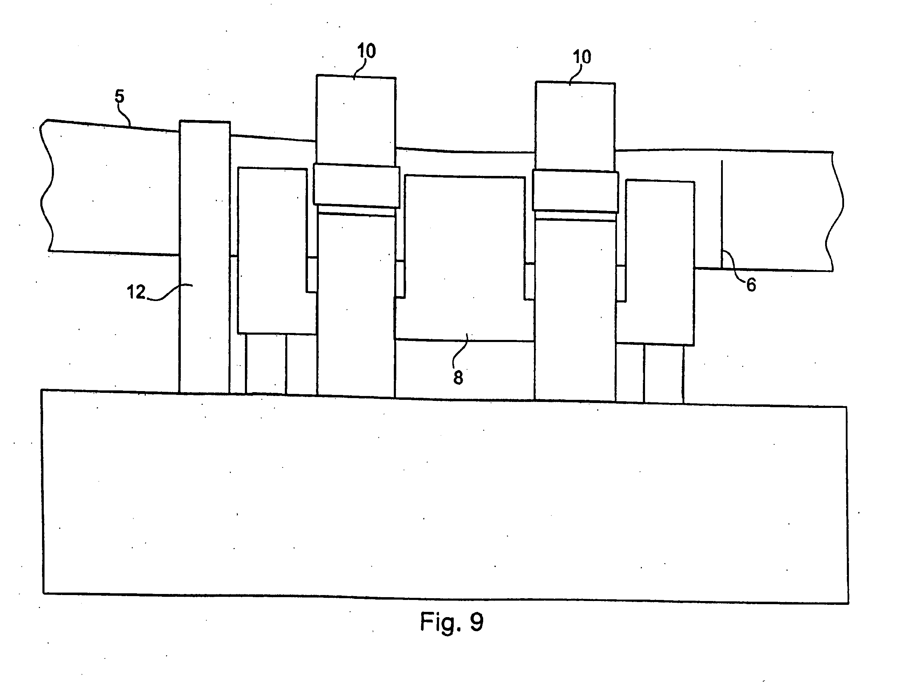

Das in Figur 1 dargestellte Stechgerät 1 bildet zusammen mit dem in den Figuren 2 bis 9 gezeigten Lanzettenvorratsband 5 ein Stechsystem. Das Lanzettenvorratsband 5 trägt mehrere Lanzetten 6, die quer zur Längsrichtung des Lanzettenvorratsbandes 5 angeordnet sind und jeweils eine Lanzettenspitze 6a zum Erzeugen einer Einstichwunde aufweisen. Die Figuren 2 bis 9 zeigen in einer schematischen Darstellung einen Ausschnitt des Geräteinneren mit einem in das Stechgerät 1 eingelegten Lanzettenvorratsband 5 in verschiedenen Betriebspositionen, anhand von denen im Folgenden die Arbeitsweise des Stechgeräts 1 erläutert wird.

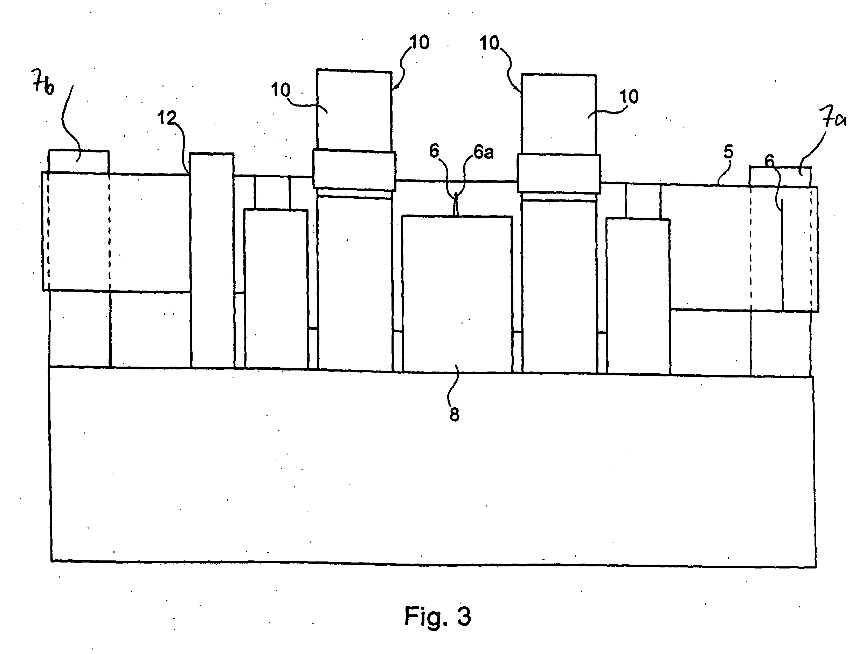

Die Lanzetten 6 eines in das Stechgerät 1 eingelegten Lanzettenvorratsbandes 5 können durch eine Fördereinrichtung nacheinander in eine Einstichposition gebracht werden. Eine Lanzette 6, die sich in der Einstichposition befindet, zeigt mit ihrer Lanzettenspitze 6a vom Inneren des Stechgeräts 1 hin zu der Geräteöffnung 2. In den Figuren 2 und 3 ist das Lanzettenvorratsband 5 so positioniert, dass sich eine Lanzette 6 in der Einstichposition befindet.

Die Fördereinrichtung kann beispielsweise eine Wickeleinrichtung sein, mit der ein Lanzettenvorratsband 5, das beispielsweise aufgewickelt oder zickzackförmig zu einem Stapel gefaltet in das Aufnahmefach des Stechgeräts 1 eingesetzt sein kann, nach Gebrauch aufgewickelt und auf diese Weise in Längsrichtung bewegt werden kann. Das Lanzettenvorratsband 5 kann beispielsweise in einer Bandkassette enthalten sein, die zum Gebrauch in das Stechgerät eingesetzt wird.

Eine Lanzette 6, die sich in Einstichposition befindet, also mit ihrer Lanzettenspitze 6a zu der Geräteöffnung 2 zeigt, wie es in Figur 2 angedeutet ist, kann mit dem Stechantrieb 8 in eine Einstichbewegung versetzt werden. Der Stechantrieb 8 umfasst bei dem dargestellten Ausführungsbeispiel eine Halterung mit einem Schlitz 9, durch den das Lanzettenvorratsband 5 hindurch geführt ist. Bei einem Einstich wird der Stechantrieb 8 mit der Halterung in Einstichrichtung bewegt und dabei der Abschnitt des Lanzettenvorratsbandes 5, der sich in dem Schlitz 9 befindet, zusammen mit einer sich in Einstichposition befindenden Lanzette 6 beschleunigt, so dass die Lanzette 6 mit ihrer Spitze 6a in ein gegen die Geräteöffnung 2 gepresstes Körperteil einsticht. Bei einem Einstich bleibt die Lanzette 6 mit dem Lanzettenvorratsband 5 verbunden, da sich bei dem Biegevorgang nur die Lanzettenspitze 6a von dem Lanzettenvorratsband 5 abhebt.

Eine Einstichbewegung der Halterung des Stechantriebs 8 kann sich im einfachsten Fall auf das Lanzettenvorratsband 5 übertragen, indem das Lanzettenvorratsband 5 mit seiner Längskante, die von den Lanzettenspitze 6a des Lanzettenvorratsbandes 5 abgewandt ist, auf der Halterung an einem Boden des Schlitzes 9 aufliegt. Eine verbesserte Übertragung einer Einstich- und Rückführbewegung des Stechantriebs 8 auf das Lanzettenvorratsband 5 kann man dadurch erreichen, dass der Schlitz 9 durch eine geeignete Mechanik verengt wird, sobald sich eine Lanzette 6 in Einstichposition befindet, und das Lanzettenvorratsband 5 auf diese Weise in dem Schlitz 9 eingeklemmt wird. Nach abgeschlossener Rückführbewegung wird der Schlitz 9 wieder verbreitert, so dass das Lanzettenvorratsband 5 von der Fördereinrichtung weiterbewegt werden kann.

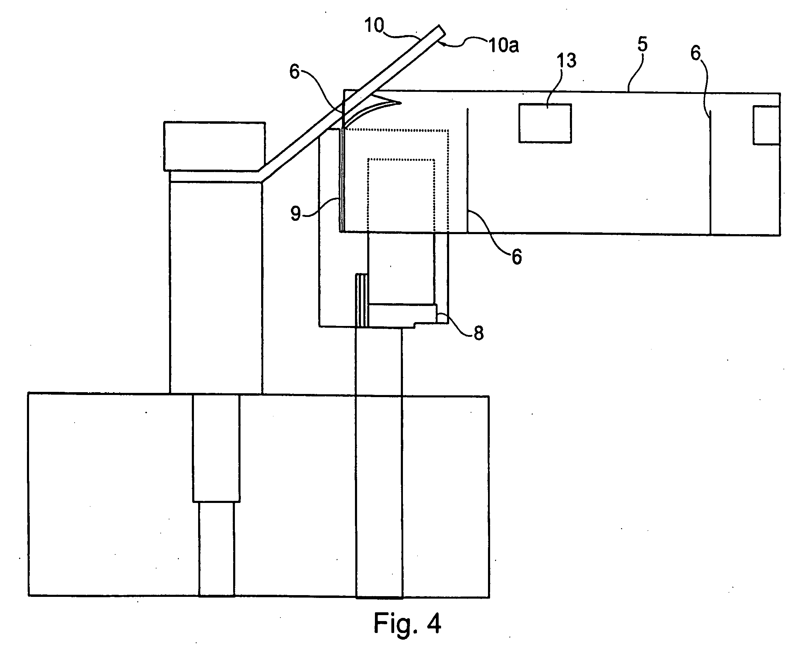

In Förderrichtung vor dem Schlitz 9 ist bei dem dargestellten Ausführungsbeispiel ein Umlenkelement 7a in Form einer Umlenkrolle angeordnet, die das Lanzettenvorratsband 5 umlenkt. Auf diese Weise kann der Transport des Lanzettenvorratsbandes 5 durch den Schlitz 9 hindurch erleichtert und ein Blockieren des Lanzettenvorratsbandes 5 in dem Schlitz 9 verhindert werden. In Förderrichtung hinter dem Schlitz 9 ist ein Umlenkelement 7b in Form einer Umlenkrolle angeordnet, die ebenso wie die Umlenkrolle 7a den Transport des Lanzettenvorratsbandes 5 durch den Schlitz 9 hindurch unterstützt. Der besseren Übersichtlichkeit wegen sind die Umlenkelemente in den Figuren 4, 5, 8 und 9 nicht dargestellt.

Die Spitzen 6a der Lanzetten 6 des eingesetzten Lanzettenvorratsbandes 5 sind von einem Sterilschutz umgeben, der bei dem dargestellten Ausführungsbeispiel als eine Kunststofffolie ausgebildet ist, welche die auf einem flexiblen Trägerband angeordneten Lanzetten 6 bedeckt. Das Trägerband kann ebenso wie der Sterilschutz beispielsweise aus Kunststoff, Papier oder Metall gefertigt werden.

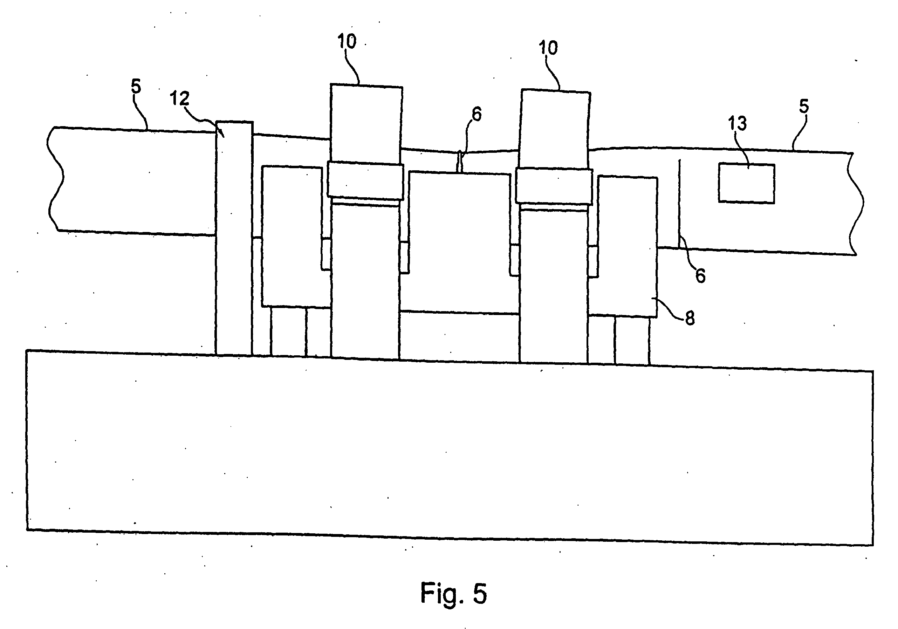

Die Lanzettenspitze 6a einer Lanzette 6 wird von dem Lanzettenvorratsband 5 getrennt, indem eine Biegeeinrichtung 10 das Lanzettenvorratsband 5 in Längsrichtung des Lanzettenvorratsbandes 5 biegt. Hiefür wird das Lanzettenvorratsband 5 von dem Stechantrieb 8 in Einstichrichtung bewegt, so dass das Lanzettenvorratsband 5 mit seiner in Einstichrichtung vorderen Längskante an die Biegeeinrichtung 10 stößt. In den Figuren 4 und 5 ist ein in Längsrichtung gebogener Abschnitt des Lanzettevorratsbandes 5 mit dem in Einstichrichtung verfahrenen Stechantrieb 8 dargestellt. Wie man darin erkennt, hat wird durch das Biegen in Längsrichtung die in Stichrichtung vordere Längskante des Lanzettenvorratsbandes 5 zu der in Stichrichtung hinteren Längskante hin gebogen, so dass der Abstand zwischen den beiden Längskanten im Vergleich zum ungebogenen Zustand etwas reduziert ist.

Bei einer derartigen Biegung des Lanzettenvorratsbandes 5 behält die Lanzette 6 ihre Orientierung bei und reißt deshalb den sie bedeckenden Sterilschutz, bei dem dargestellten Ausführungsbeispiel eine Deckfolie, oder das flexible Trägerband auf. Die Lanzettenspitze 6a hebt sich bei dem Biegevorgang von dem Lanzettenvorratsband 5 ab und wird auf diese Weise von ihm getrennt, so dass mit der Lanzettenspitze 6a zum Gewinnen einer Körperflüssigkeitsprobe in ein gegen die Geräteöffnung 2 gepresstes Körperteil eingestochen werden kann.

Die Biegeeinrichtung 10 wirkt zum Biegen des Lanzettenvorratsbandes 5 auf eine Längskante des Lanzettenvorratsbandes 5 ein. Bei dem dargestellten Ausführungsbeispiel wird das Lanzettenvorratsband 5 aus der in den Figuren 2 und 3 dargestellten Position in Einstichrichtung bewegt, bis es in die in den Figuren 4 und 5 dargestellte Position gelangt, in der ein Abschnitt des Lanzettenvorratsbands 5 in Längsrichtung gebogen wird, indem eine Längskante des Lanzettenvorratsbandes 5 von dem Stechantrieb 8 gegen die Biegeeinrichtung 10 gedrückt wird. Das Biegen des Lanzettenvorratsbandes 5 in Längsrichtung und damit das Bereitmachen einer Lanzette 6 für einen Einstich erfolgt bei dem dargestellten Ausführungsbeispiel in einer frühen Phase einer Einstichbewegung. Auf diese Weise kann die Gefahr einer Verschmutzung der Lanzettenspitze 6a, die nach dem Trennen der Lanzettenspitze 6a von dem Lanzettenvorratsband 5 prinzipiell wegen des Durchbrochenen Sterilschutzes besteht, minimiert.

Prinzipiell ist es ausreichend, mit einer Biegeeinrichtung 10 an einer Stelle, beispielsweise neben einer Lanzette 6 auf eine Längskante des Lanzettenvorratsbandes 5 einzuwirken. Eine bessere Biegung lässt sich jedoch erreichen, wenn die Biegeeinrichtung 10 an zwei voneinander beabstandeten Stellen auf die den Lanzettenspitzen 6a zugewandte Längskante des Lanzettenvorratsbandes 5 einwirkt, so dass zwischen diesen beiden Stellen ein Bandschnitt gebogen wird, der keinen Kontakt zu der Biegeeinrichtung 10 hat und eine Lanzettenspitze 6a, die sich in diesem Abschnitt des Lanzettenvorratsbandes 5 befindet, die Biegeeinrichtung 10 nicht berührt. Dies lässt sich beispielsweise dadurch erreichen, dass die Biegeeinrichtung 10 einen Schlitz aufweist. Auf diese Weise kann eine Lanzettenspitze 6a beim Biegen des Lanzettenvorratsbandes 5 ihre Orientierung beibehalten und in den Schlitz hineinragen. In Figur 5 ist dargestellt, wie die Biegeeinrichtung 10 links und rechts neben der Lanzette 6 auf eine Längskante des Lanzettenvorratsbandes 5 drückt.

Bei dem dargestellten Ausführungsbeispiel hat die Biegeeinrichtung 10 zwei Biegeflächen 10a, die sich in einem Abstand voneinander schräg zur Einstichrichtung erstrecken. Wie insbesondere die Figuren 2 und 4 zeigen, können derartige Biegeflächen 10a gabelartig, beispielsweise als Stege, ausgebildet sein, die bei dem dargestellten Ausführungsbeispiel geradlinig verlaufen. Prinzipiell können die Biegeflächen 10a jedoch auch gekrümmt sein. Es genügt, wenn eine Längskante des Lanzettenvorratsbandes 5, die gegen eine Biegefläche 10a gedrückt wird, abgleitet, so dass der entsprechende Abschnitt des Lanzettenvorratsbandes 5 in Längsrichtung abgebogen wird. Beim Biegen eines Abschnitts des Lanzettenvorratsbandes 5 in Längsrichtung bildet sich eine Wölbung, die sich in Längsrichtung erstreckt und in den Figuren 4 und 5 zu erkennen ist.

Das in Figur 1 gezeigte Ausführungsbeispiel weist eine Testeinrichtung 12 zum Untersuchen einer Körperflüssigkeitsprobe auf. Eine derartige Testeinrichtung 12 kann eine photometrische Testeinrichtung sein, mit der eine Verfärbung eines Testfeldes untersucht wird, das Nachweisreagenzien enthält, die eine von der Analytkonzentration abhängende Verfärbung des Testfeldes bewirken. Beispielsweise kann die Testeinrichtung 12 auch eine elektrochemische Testeiririchtung sein, mit der ein von der Analytkonzentration abhängender Messstrom erfasst wird. Geeignete Testeinrichtungen sind beispielsweise in handelsübliche Analysehandgeräte integriert, mit denen beispielsweise Diabetiker ihren Blutzuckergehalt überwachen. Das Lanzettenvorratsband 5 trägt neben Lanzetten 6 auch Testfelder 13, so dass mit dem beschriebenen Stechsystem eine Körperflüssigkeitsprobe gewonnen und anschließend, beispielsweise zur Messung des Glukosegehalts, untersucht werden kann.

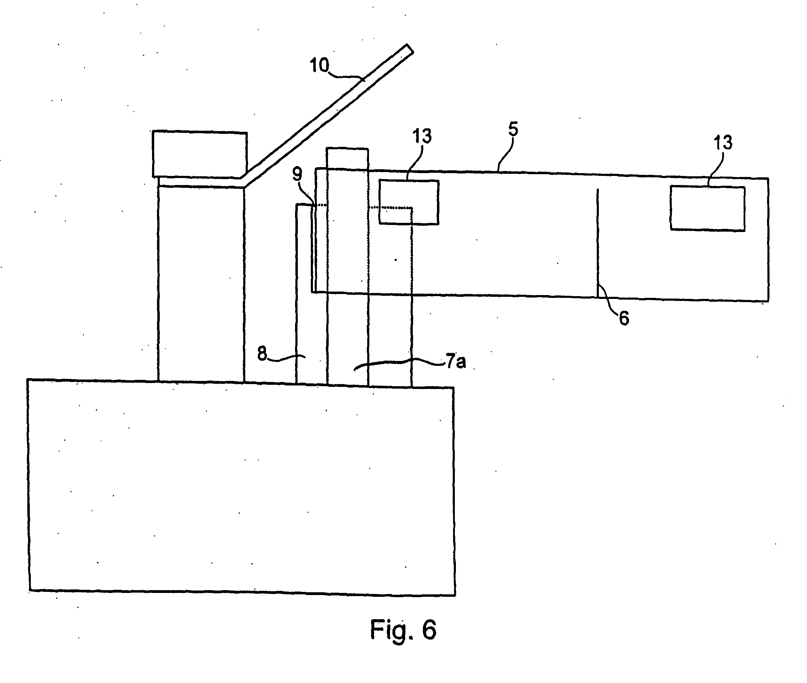

Die Testeinrichtung 12 des Stechgeräts 1 wirkt mit einem Testfeld 13 des Lanzettenvorratsbandes 5 zusammen, das eine aus einer erzeugten Einstichwunde austretende Körperflüssigkeitsprobe aufnimmt. Dies wird im Folgenden anhand der Figuren 6 bis 9 erläutert.

Die mit einer Einstichbewegung begonnene Einwirkung des Biegemechanismus 10 auf das Lanzettenvorratsband 5 wird durch eine anschließende Rückführbewegung beendet, bei welcher der Lanzettenantrieb 8 und der in dem Schlitz 9 angeordnete Abschnitt des Lanzettenvorratsbandes 5 wieder in ihre in den Figuren 2 und 6 dargestellte Ausgangsposition zurückkehren, so dass die Biegeeinrichtung 10 nicht mehr gegen das Lanzettenvorratsband 5 drückt. Zum Aufnehmen einer Körperflüssigkeitsprobe wird so die Einwirkung des Biegemechanismus 10 auf das Lanzettenvorratsband 5 nach einem Einstich unterbrochen, so dass der gebogene Lanzettenvorratsbandabschnitt wieder in seine flache Lage zurückkehrt, in der er beispielsweise in Figur 6 dargestellt ist.

Anschließend wird das Lanzettenvorratsband 5 von der Fördereinrichtung weiterbewegt, so dass ein Testfeld 13 des Lanzettenvorratsbandes 5 mit der Geräteöffnung 2 und einer zuvor erzeugten Einstichwunde eines gegen die Geräteöffnung 2 gepressten Körperteils fluchtet. Danach wird das Lanzettenvorratsband 5 von dem Stechantrieb 8 erneut in Einstichrichtung bewegt und so von dem Biegemechanismus 10 erneut in Längsrichtung gebogen. Dabei wird das Lanzettenvorratsband 5 mit einer Teilfläche an das Körperteil angelegt, in dem die Einstichwunde erzeugt wurde. Bei dem dargestellten Ausführungsbeispiel enthält die Teilfläche, mit der das Lanzettenvorratsband 5 an das Körperteil angelegt wird, ein Testfeld 13. Das Lanzettenvorratsband 5 wird in diesem Arbeitsschritt also erneut mit seiner Längskante gegen die Biegeeinrichtung 10 gedrückt.

Indem eine Körperflüssigkeitsprobe durch Anlegen einer Teilfläche des in Längsrichtung gebogenen Lanzettenvorratsbandes an ein Körperteil, in dem eine Einstichwunde erzeugt wurde, aufgenommen wird, kann eine Verschmutzung des Stechgeräts vorteilhaft vermieden werden. Verwendete Lanzetten und untersuchte Körperflüssigkeitsproben können zusammen mit einem aufgebrauchten Lanzettenvorratsband hygienisch und benutzerfreundliche entsorgt werden.

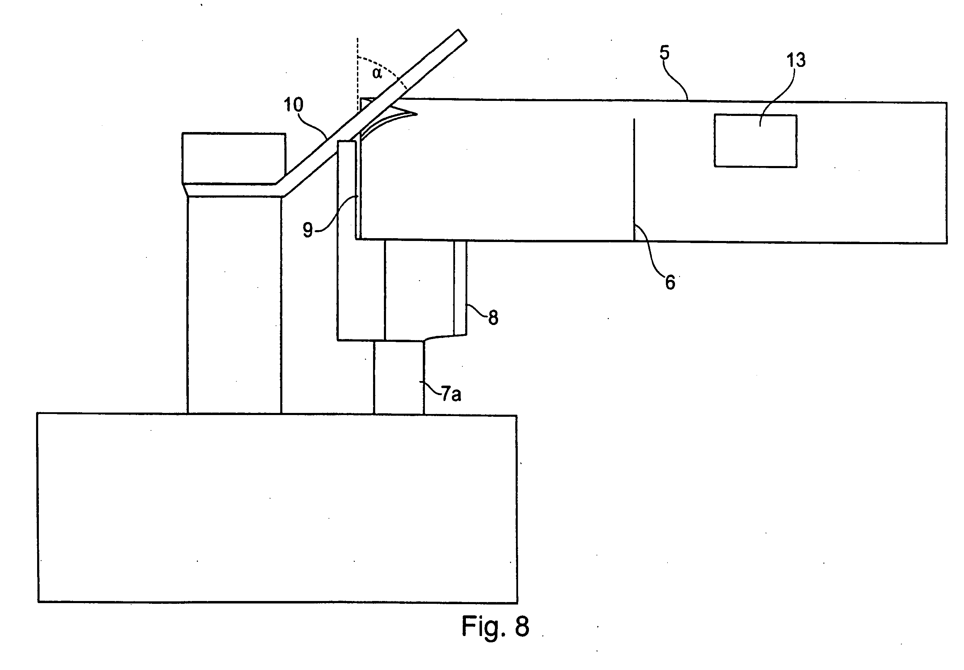

Von der Biegeeinrichtung 10 wird das Lanzettenvorratsband 5 bei dem dargestellten Ausführungsbeispiel um einen in Figur 8 dargestellten Winkel α von etwa 45° in Längsrichtung gebogen. Das Lanzettenvorratsband wird bei dem dargestellten Ausführungsbeispiel um mindestens 25°, insbesondere mindestens 30° gebogen. Besonders günstig zur Aufnahme einer Körperflüssigkeitsprobe sind Biegewinkel von 40° bis 90°, so dass eine vorteilhaft große Teilfläche des Lanzettenvorratsbandes 5 zum Aufnehmen einer Körperflüssigkeitsprobe an das betreffende Körperteil angelegt werden kann.

Nach dem Aufnehmen einer Körperflüssigkeitsprobe wird das Lanzettenvorratsband 5 von dem Stechantrieb 8 wieder zurückbewegt, also erneut von der Biegeeinrichtung 10 getrennt und somit die Einwirkung der Biegeeinrichtung 10 erneut unterbrochen, so dass der gebogene Lanzettenvorratsbandabschnitt wieder in seine flache Lage zurückkehrt. Anschließend wird das Lanzettenvorratsband 5 von der Fördereinrichtung einen Schritt weiter transportiert, um das mit einer Körperflüssigkeitsprobe benetzte Testfeld 13 zu der Testeinrichtung 12 zu befördern. Die Testeinrichtung 12 ermittelt dann in Zusammenwirkung mit dem Testfeld 13 eine Analytkonzentration, beispielsweise die Glukosekonzentration, der Körperflüssigkeit.

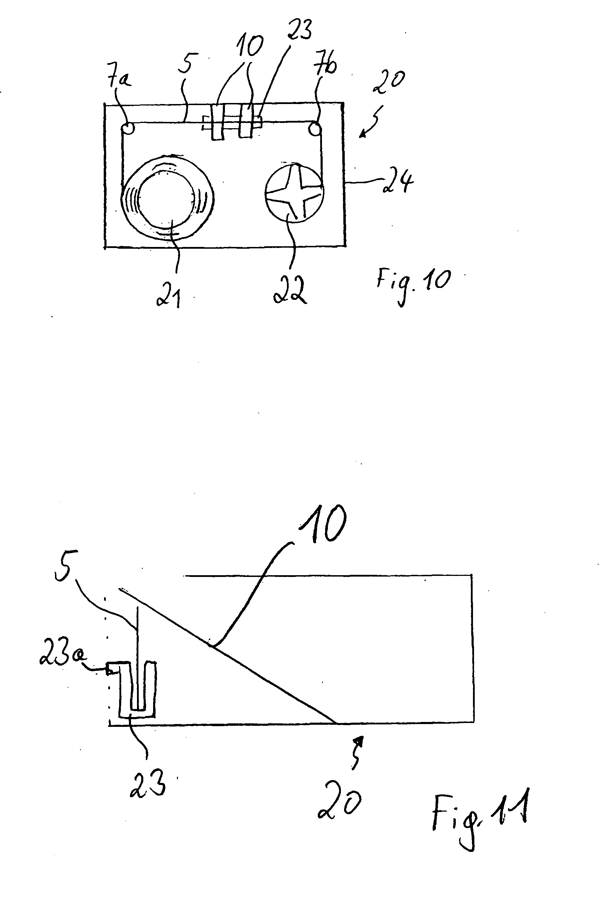

Figur 10 zeigt eine schematische Darstellung eines Ausführungsbeispiels einer Bandkassette 20 mit einem Lanzettenvorratsband 5, das mehrere Lanzetten trägt, die quer zur Längsrichtung des Lanzettenvorratsbands 5 angeordnet sind. Die Bandkassette hat eine Biegeeinrichtung 10, um einen Abschnitt des Lanzettenvorratsbandes 5 in Längsrichtung des Lanzettenvorratsbandes zu biegen und dadurch eine Lanzettenspitze einer Lanzette für einen Einstich von dem Lanzettenvorratsband 5 zu trennen.

Alle Ausführungen zur Biegeeinrichtung 10 des vorstehend anhand der Figuren 1 bis 9 beschriebenen Ausführungsbeispiels und den Biegevorgang lassen sich auf das in Figur 10 dargestellte Ausführungsbeispiel übertragen. Die Biegeeinrichtung 10 ist ebenso wie die Biegeeinrichtung des vorstehend beschriebenen Ausführungsbeispiels als zwei schräg zur Stichrichtung verlaufende Stege ausgebildet. Bei dem dargestellten Ausführungsbeispiel ist die Biegeeinrichtung an einem Rahmen 24 der Bandkassette 20 befestigt, was am besten in Figur 11, einer schematischen Seitenansicht zu Figur 10, zu erkennen ist.

Der Rahmen 24 der Bandkassette trägt eine Vorratsrolle 21, auf der das Lanzettenvorratsband mit unbenutzten Lanzetten aufgewickelt ist, und eine Förderrolle 22, auf die benutzte Abschnitte des Lanzettenvorratsbands 5 aufgewickelt werden. Die Förderrolle 22 hat eine zentrische Ausnehmung mit einer von der Kreisform abweichenden Kontur, bei der dargestellten Ausführung eine sternförmige Kontur, um beim Einsetzen der Bandkassette 20 in ein Stechgerät 1 an eine angetriebene Welle des Stechgeräts 1 anzukoppeln.

Das Lanzettenvorratsband 5 wird in der Bandkassette 20 mittels Umlenkrollen 7a, 7b durch einen Kopplungskopf 23 hindurchgeführt. Der Kopplungskopf 23 koppelt beim Einsetzen der Bandkassette 20 in ein Stechgerät 1 an den Stechantrieb 8 des Stechgeräts 1 an, so dass der Kopplungskopf 23 in Stichrichtung bewegt werden kann. Bei dem dargestellten Ausführungsbeispiel hat der Kopplungskopf 23 zum Ankoppeln an den Stechantrieb 8 eines Stechgeräts 1 ein Kopplungselement 23a in Form eines Zapfens. Der Kopplungskopf 23 hat einen Schlitz oder eine Nut, durch die das Lanzettenvorratsband 5 hindurchgeführt ist. Bei einer Hubbewegung des Kopplungskopfs 23 wird das Lanzettenvorratsband 5 in Stichrichtung mitbewegt, so dass seine Längskante gegen die Biegeeinrichtung 10 trifft und das Lanzettenvorratsband abgebogen wird. Bei diesem Biegen des Lanzettenvorratsbands 5 löst sich die Spitze der Lanzette 6 für einen Einstich von dem Lanzettenvorratsband 5 ab.

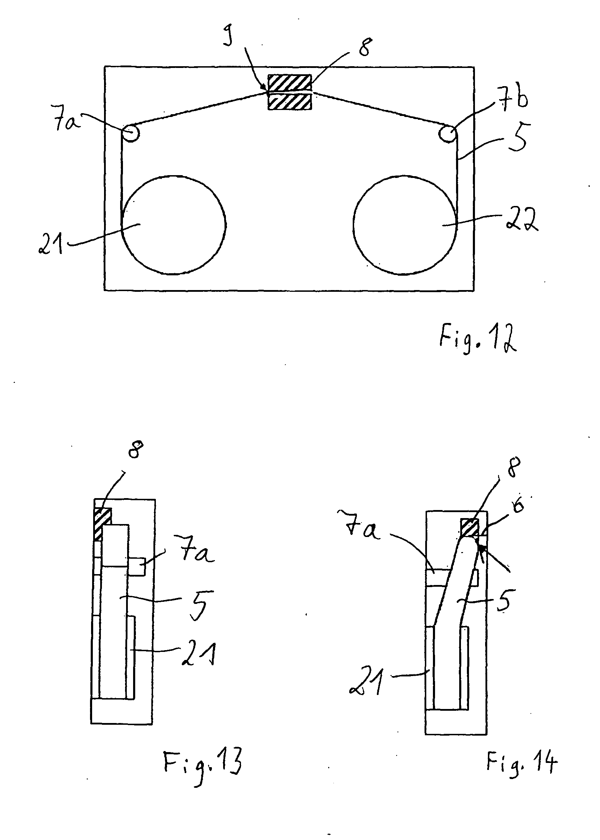

Figur 12 zeigt in einer schematischen Darstellung eine Draufsicht eines weiteren Ausführungsbeispiels eines Stechgeräts oder Stechsystems, bei dem das Lanzettenvorratsband 5 in Längsrichtung gebogen wird, so dass sich die Lanzettenspitze einer Lanzette 6 von dem Lanzettenvorratsband 5 abhebt. Im Unterschied zu den vorstehend beschriebenen Ausführungsbeispielen wird zum Biegen des Lanzettenvorratsbandes 5 aber nicht auf dessen Längskante eingewirkt. Stattdessen wird die Biegung dadurch erreicht, dass bei einer Stichbewegung die in Stichrichtung vordere Längskante des Lanzettenvorratsbandes 5 einer größeren Spannung als die in Stichrichtung hintere Längskante ausgesetzt wird. Dies wird im Folgenden unter Bezugnahme auf die Figuren 12 bis 14 erläutert.

Bei dem in Figur 12 schematisch in einer Draufsicht und in den Figuren 13 und 14 vor bzw. während einer Stichbewegung dargestellten Ausführungsbeispiel erfolgt die Stichbewegung in Richtung der geometrischen Drehachse der Förderrolle 22. Ähnlich wie bei den vorstehend beschriebenen Ausführungsbeispielen ist eine Lanzette 6 in der Einstichposition zusammen mit einem sie tragenden Abschnitt des Lanzettenvorratsbandes 5 in einer Halterung 8 des Stechantriebs gehalten. Die Halterung 8 hat einen Schlitz 9, durch den das Lanzettenvorratsband 5 hindurchgeführt ist. Die Halterung 8 ist ebenso wie bei den vorstehend beschriebenen Ausführungsbeispielen zwischen zwei Umlenkelementen 7a und 7b, beispielsweise Zapfen, angeordnet, an denen das Lanzettenvorratsband 5 vorbeigeführt wird und die es dabei umlenken, also seine Bewegungsrichtung ändern, in Figur 12 um etwas mehr als 90°. Im Unterschied zu den vorstehend beschriebenen Ausführungsbeispielen ist die Halterung 8 aber nicht in einer Linie mit den Umlenkelementen 7a, 7b angeordnet, sondern quer zur Stichrichtung versetzt. Der zu der Halterung 8 hinführende Abschnitt des Lanzettenvorratsbandes 5 schließt deshalb mit dem von der Halterung 8 wegführenden Abschnitt des Lanzettenvorratsbandes 5 einen Winkel von weniger als 180° ein.

Bei einem Stich bewegt sich die Halterung 8 aus der in Figur 13 dargestellten Ausgangsposition in die in Figur 14 dargestellte Position. Dabei wird der von der Halterung 8 gehaltene Bandabschnitt aus der Wickelebene, in der das Lanzettenvorratsband 5 von der Förderrolle 22 aufgewickelt wird, herausbewegt. Dadurch, dass die Halterung 8 quer zur Stichrichtung versetzt zwischen den beiden Umlenkelementen 7a und 7b angeordnet ist, wird bei dieser Bewegung die in Stichrichtung vordere Längskante des Lanzettenvorratsbandes 5 einer Zugspannung ausgesetzt. Diese Zugspannung bewirkt, dass sich die in Stichrichtung vordere Längskante zu den Umlenkelementen 7a, 7b hinbiegt. Die umgebogene Längskante des Lanzettenvorratsbandes 5 ist in Figur 14 durch einen Pfeil angedeutet. Auf diese Weise wird bei dem in den Figuren 12 bis 14 dargestellten Ausführungsbeispiel eine Biegung des Lanzettenvorratsbandes 5 in Längsrichtung auch ohne Einwirken auf die zu biegende Längskante erzielt.

Die Biegeeinrichtung ist bei dem in den Figuren 12 bis 14 dargestellten Ausführungsbeispiel also dadurch gebildet, dass eine Lanzette 6 in der Einstichposition zusammen mit einem sie tragenden Abschnitt des Lanzettenvorratsbandes 5 in der Halterung 8 des Stechantriebs gehalten ist, die zwischen zwei Umlenkelementen 7a, 7b, an denen das Lanzettenvorratsband 5 vorbeigeführt ist, quer zur Stichrichtung versetzt angeordnet ist und bei einem Stich eine Bewegung in Stichrichtung ausführt.

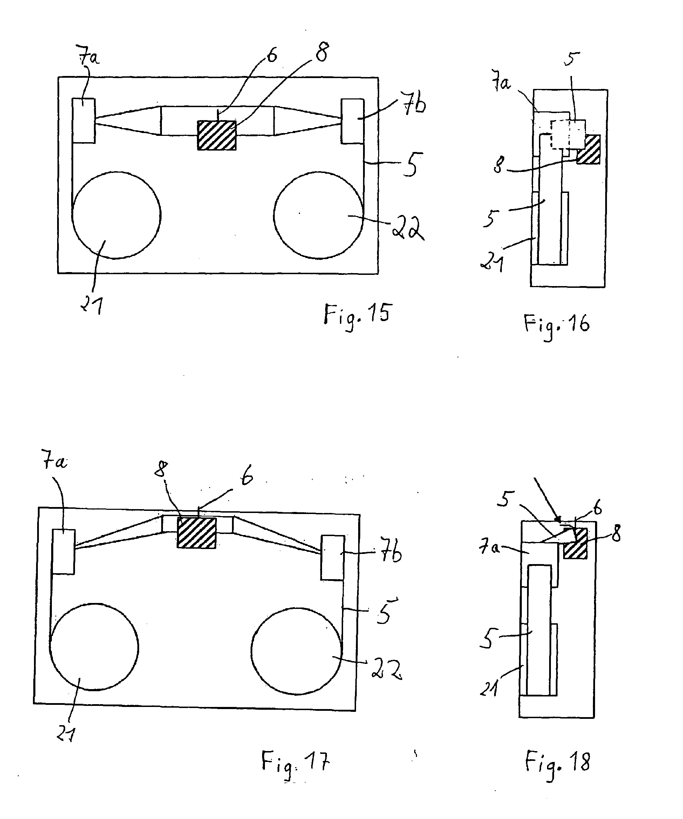

In den Figuren 15 bis 18 ist ein weiteres Ausführungsbeispiel dargestellt, bei dem ähnlich wie bei dem vorstehend beschriebenen Ausführungsbeispiel eine Längsbiegung des Lanzettenvorratsbandes 5 erreicht wird, ohne dass auf die in Stichrichtung vordere Längskante des Lanzettenvorratsbandes 5 eingewirkt wird.

Figur 15 zeigt eine schematische Draufsicht eines Stechgeräts oder Stechsystems vor einer Stichbewegung. Der Unterschied zu dem vorstehend beschriebenen Ausführungsbeispiel besteht dabei im wesentlichen darin, dass die Stichrichtung quer zur geometrischen Drehachse der Förderrolle 22 verläuft. Das Lanzettenvorratsband 5 wird deshalb auf seinem Weg von der Vorratsrolle 21 zu der Halterung 8 des Stechantriebs um eine Vierteldrehung gedreht. Von der Halterung 8 zu der Förderrolle 22 erfolgt eine weitere Vierteldrehung. Auf diese Weise verläuft die Ebene des Lanzettenvorratsbandes 5 in der Halterung 8 quer zur Ebene des Lanzettenvorratsbandes 5 im aufgewickelten Zustand.

Die Vierteldrehung des Lanzettenvorratsbandes 5 erfolgt bei dem dargestellten Ausführungsbeispiel an den Umlenkelementen 7a, 7b, an denen das Lanzettenvorratsband 5 auf seinem Weg zur Förderrolle 22 vorbeigeführt wird und dabei auch seine Bewegungsrichtung ändert, bei dem dargestellten Ausführungsbeispiel um etwa 90°. Ähnlich wie bei dem vorstehend beschriebenen Ausführungsbeispiel ist die Halterung 8 zwischen den beiden Umlenkelementen 7a, 7b quer zur Stichrichtung und quer zu einer gedachten Verbindungslinie der beiden Umlenkelemente 7a, 7b versetzt angeordnet. Dies ist in Figur 16 deutlich daran zu erkennen, dass die Halterung 8 dort rechts von der Vorratsrolle 21 und dem Umlenkelement 7a dargestellt ist, sich also in der Figur 15 "über" den beiden Umlenkelementen 7a, 7b befindet. Auch bei dem in den Figuren 15 bis 18 dargestellten Ausführungsbeispiel schließt deshalb der in Transportrichtung des Bandes zu der Halterung 8 hinführende Abschnitt des Lanzettenvorratsbandes 5 mit dem von der Halterung 8 wegführenden Abschnitt des Lanzettenvorratsbandes 5 einen Winkel von weniger als 180° ein.

Ebenso wie bei dem unter Bezugnahme auf die Figuren 12 bis 14 beschriebenen Ausführungsbeispiel bewirkt deshalb auch bei dem in den Figuren 15 bis 18 dargestellten Ausführungsbeispiel bei einer Stichbewegung der Halterung 8 auf die in Stichrichtung vordere Längskante des Lanzettenvorratsbandes 5 eine Zugspannung. Dieser Zugspannung gibt die vordere Längskante des Lanzettenvorratsbandes 5 nach, indem sie sich zu der in Stichrichtung hinteren Längskante hinbiegt. Bei einer Stichbewegung wird so auch bei diesem Ausführungsbeispiel das Lanzettenvorratsband 5 in Längsrichtung gebogen, so dass sich die Lanzettenspitze einer Lanzette 6, die sich in der Einstichposition befindet, von dem Lanzettenvorratsband 5 abhebt. In Figur 18 ist ein Pfeil eingezeichnet, der auf die abgebogene Längskante des Lanzettenvorratsbandes 5 deutet.

Auch bei dem in den Figuren 15 bis 18 dargestellten Ausführungsbeispiel ist die Biegeeinrichtung auf diese Weise durch die besondere Bandführung, also die Anordnung der Halterung 8 in Bezug auf die Umlenkelemente 7a, 7b ausgebildet.

### Bezugszeichenliste

- 1: Stechgerät
- 2: Geräteöffnung
- 3: Bedienungselemente
- 4: Zeigeeinrichtung
- 5: Lanzettenvorratsband
- 6: Lanzetten
- 6a: Lanzettenspitze
- 7a: Umlenkelement
- 7b: Umlenkelement
- 8: Stechantrieb
- 9: Schlitz
- 10: Biegeeinrichtung
- 10a: Biegefläche

- 12: Testeinrichtung
- 13: Testfeld

- 20: Bandkassette
- 21: Vorratsrolle
- 22: Förderrolle
- 23: Kopplungskopf
- 23a: Kopplungselement
- 24: Rahmen

## Patentansprüche

1. Stechsystem zum Gewinnen einer Körperflüssigkeitsprobe umfassend ein Lanzettenvorratsband (5) mit mehreren Lanzetten (6), die quer zur Längsrichtung des Lanzettenvorratsbandes (5) angeordnet sind,
einer Fördereinrichtung, um durch Transportieren eines in das Aufnahmefach eingesetzten Lanzettenvorratsbandes (5) die Lanzetten (6) des Lanzettenvorratsbands (5) nacheinander in eine Einstichposition zu bringen, und
einem Stechantrieb (8), um eine Lanzette (6), die sich in der Einstichposition befindet, in eine Einstichbewegung zu versetzten,
**gekennzeichnet durch**
eine Biegeeinrichtung (10), um einen Abschnitt des Lanzettenvorratsbands (5) in Längsrichtung des Lanzettenvorratsbandes (5) zu biegen und **dadurch** eine Lanzettenspitze (6a) einer Lanzette (6) für einen Einstich von dem Lanzettenvorratsband (5) zu trennen.

2. Stechsystem nach Anspruch 1, **gekennzeichnet durch**
eine Bandkassette mit einem Lanzettenvorratsband (5) mit mehreren Lanzetten (6), die quer zur Längsrichtung des Lanzettenvorratsbandes (5) angeordnet sind, und
ein Stechgerät mit
einem Aufnahmefach zum Aufnehmen einer Bandkassette,
einer Fördereinrichtung, um **durch** Transportieren des Lanzettenvorratsbandes (5) einer in das Aufnahmefach eingesetzten Bandkassette die Lanzetten (6) des Lanzettenvorratsbands (5) nacheinander in eine Einstichposition zu bringen, und einem Stechantrieb (8), um eine Lanzette (6), die sich in der Einstichposition befindet, in eine Einstichbewegung zu versetzten.

3. Stechsystem nach einem der vorstehenden Ansprüche, **dadurch gekennzeichnet, dass** die Lanzettenspitzen (6a) der Lanzetten (6) des Lanzettenvorratsbandes (5) von einem Sterilschutz umgeben sind, der bei dem Biegen eines eine Lanzette (6) enthaltenden Abschnitts des Lanzettenvorratsbandes (5) von einer Lanzettenspitze (6a) durchbrochen wird.

4. Bandkassette mit
einem Lanzettenvorratsband (5) mit mehreren Lanzetten (6), die quer zur Längsrichtung des Lanzettenvorratsbandes (5) angeordnet sind, **gekennzeichnet, durch** eine Biegeeinrichtung (10), um einen Abschnitt des Lanzettenvorratsbands (5) in Längsrichtung des Lanzettenvorratsbandes (5) zu biegen und **dadurch** eine Lanzettenspitze (6a) einer Lanzette (6) für einen Einstich von dem Lanzettenvorratsband (5) zu trennen.

5. Stechgerät zum Gewinnen einer Körperflüssigkeitsprobe mit einem Aufnahmefach zum Aufnehmen eines Lanzettenvorratsbands (5) mit mehreren Lanzetten (6), die quer zur Längsrichtung des Lanzettenvorratsbandes (5) angeordnet sind und eine Lanzettenspitze (6a) zum Erzeugen einer Einstichwunde aufweisen,
einer Fördereinrichtung, um durch Transportieren eines in das Aufnahmefach eingesetzten Lanzettenvorratsbandes (5) die Lanzetten (6) des Lanzettenvorratsbands (5) nacheinander in eine Einstichposition zu bringen, und
einem Stechantrieb (8), um eine Lanzette (6), die sich in der Einstichposition befindet, in eine Einstichbewegung zu versetzten,
**gekennzeichnet durch**
eine Biegeeinrichtung (10), um einen Abschnitt des Lanzettenvorratsbands (5) in Längsrichtung des Lanzettenvorratsbandes (5) zu biegen und **dadurch** eine Lanzettenspitze (6a) einer Lanzette (6) für einen Einstich von dem Lanzettenvorratsband (5) zu trennen.

6. Stechgerät nach Anspruch 5, **dadurch gekennzeichnet, dass** die Biegeeinrichtung (10) zum Biegen des Lanzettenvorratsbandes (5) auf eine Längskante des Lanzettenvorratsbandes (5) einwirkt.

7. Stechgerät nach Anspruch 6, **dadurch gekennzeichnet, dass** die Biegeeinrichtung (10) zum Biegen des Lanzettenvorratsbandes (5) mit mindestens einer sich schräg zur Einstichrichtung erstreckenden Biegefläche (10a) auf eine Längskante des Lanzettenvorratsbandes (5) einwirkt.

8. Stechgerät nach einem der Ansprüche 5 bis 7, **dadurch gekennzeichnet, dass** der zu biegende Abschnitt des Lanzettenvorratsbands (5) zum Biegen in Einstichrichtung bewegt wird.

9. Stechgerät nach einem der Ansprüche 5 bis 8, **dadurch gekennzeichnet, dass** der Stechantrieb (8) für einen Einstich einen Abschnitt des Lanzettenvorratsbandes (5) zusammen mit einer Lanzette (6) in Einstichrichtung bewegt.

10. Stechgerät nach einem der Ansprüche 5 bis 9, **dadurch gekennzeichnet, dass** das Lanzettenvorratsband (5) austauschbar in dem Aufnahmefach angeordnet ist.

11. Stechgerät nach einem der Ansprüche 5 bis 10, **gekennzeichnet durch** eine auswechselbar in dem Aufnahmefach angeordnete Bandkassette, die das das Lanzettenvorratsband (5) enthält.

12. Stechgerät nach Anspruch 11, **dadurch gekennzeichnet, dass** die Biegeeinrichtung (10) an der Bandkassette angebracht ist.

13. Stechgerät nach einem der Ansprüche 5 bis 12, **dadurch gekennzeichnet, dass** die Biegeeinrichtung **dadurch** gebildet ist, dass eine Lanzette (6) in der Einstichposition zusammen mit einem sie tragenden Abschnitt des Lanzettenvorratsbandes (5) in einer Halterung (8) des Stechantriebs gehalten ist, die zwischen zwei Umlenkelementen (7a, 7b), an denen das Lanzettenvorratsband (5) vorbeigeführt ist, quer zur Stichrichtung versetzt angeordnet ist und bei eine Stich eine Bewegung in Stichrichtung ausführt.

14. Verfahren zum Trennen einer Lanzettenspitze (6a) von einem Lanzettenvorratsband (5), indem das Lanzettenvorratsband (5) in einem Stechgerät in Längsrichtung gebogen wird, so dass sich die Lanzettenspitze (6a) von dem Lanzettenvorratsband (5) abhebt.

15. Verfahren nach Anspruch 14, **dadurch gekennzeichnet, dass** zum Biegen des Bandes (5) eine in einem Stechgerät angeordneten Biegeeinrichtung (10) verwendet wird.

## Claims

1. A lancing system for collecting samples of a body liquid comprising a lancet supply strip (5) with a plurality of lancets (6), arranged transversely to the lengthwise direction of the lancet supply strip (5);
a feed mechanism for transporting a lancet supply strip (5), placed in the receptacle, to move the lancets (6) of a lancet supply strip (5) one after the other to a puncturing position; and
a lancing drive (8) for causing a lancet (6), which occupies a puncturing position, to perform a puncturing movement,
**characterized by**
a bending mechanism (10) for bending a section of the lancet supply strip (5) in lengthwise direction of the lancet supply strip (5) and for thereby separating a lancet tip (6a) of a lancet (6) from the lancet supply strip (5) for a puncturing action.

2. The lancing system as defined in Claim 1, **characterized by** a tape cassette comprising a lancet supply strip (5) with a plurality of lancets (6) arranged transversely to the lengthwise direction of the lancet supply strip (5); and
a lancing device having
a receptacle for accommodating a cassette;
a feed mechanism for transporting the lancet supply strip (5) of a cassette, placed in the receptacle, to move the lancets (6) of the lancet supply strip (5) one after the other to a puncturing position; and
a lancing drive (8) for causing a lancet (6), which occupies a puncturing position, to perform a puncturing movement.

3. The lancing system as defined in any of the preceding claims, **characterized in that** the lancet tips (6a) of the lancets (6) of the lancet supply strip (5) are enclosed by a sterility protection which is broken by a lancet tip (6a) when a section of the lancet supply strip (5) containing a lancet (6) is bent.

4. A cassette having
a lancet supply strip (5) with a plurality of lancets (6), arranged transversely to the lengthwise direction of the lancet supply strip (5), **characterized by** a bending mechanism (10) for bending a section of the lancet supply strip (5) in lengthwise direction of the lancet supply strip (5) and for thereby separating a lancet tip (6a) of a lancet (6) from the lancet supply strip (5) for a puncturing action.

5. A lancing device for collecting a sample of a body liquid having a receptacle for accommodating a lancet supply strip (5) with a plurality of lancets (6), arranged transversely to the lengthwise direction of the lancet supply strip (5) and provided with a lancet tip (6a) for producing a puncturing wound;
a feed mechanism for transporting a lancet supply strip (5), placed in the receptacle, to move the lancets (6) of a lancet supply strip (5) one after the other to a puncturing position; and
a lancing drive (8) for causing a lancet (6), which occupies a puncturing position, to perform a puncturing movement,
**characterized by**
a bending mechanism (10) for bending a section of the lancet supply strip (5) in lengthwise direction of the lancet supply strip (5) and for thereby separating a lancet tip (6a) of a lancet (6) from the lancet supply strip (5) for a puncturing action.

6. The lancing device as defined in Claim 5, **characterized in that** for bending the lancet supply strip (5) the bending mechanism (10) acts on a longitudinal edge of the lancet supply strip (5).

7. The lancing device as defined in Claim 6, **characterized in that** for bending the lancet supply strip (5) the bending mechanism (10) acts on a longitudinal edge of the lancet supply strip (5) by at least one bending surface (10a) extending obliquely to the puncturing direction.

8. The lancing device as defined in any of claim 5 to 7, **characterized in that** the section of the lancet supply strip (5) to be bent is moved in the puncturing direction for bending.

9. The lancing device as defined in any of claim 5 to 8, **characterized in that** for performing a puncturing action, the lancing drive (8) moves a section of the lancet supply strip (5), together with a lancet (6), in the puncturing direction.

10. The lancing device as defined in any of claim 5 to 9, **characterized in that** the lancet supply strip (5) is exchangeably arranged in a receptacle.

11. The lancing device as defined in any of claim 5 to 10, **characterized by** a strip cassette, arranged exchangeably in the receptacle, that contains the lancet supply strip (5).

12. The lancing device as defined in any of the preceding claims, **characterized in that** the bending mechanism (10) is attached to the strip cassette.

13. The lancing device as defined in any of claim 5 to 12, **characterized in that** the bending mechanism is designed **in that** a lancet (6), together with the section of the lancet supply strip (5) on which it is carried, is held in a holder (8) of the lancet drive, the holder being arranged transversely offset to the puncturing direction between two guide elements (7a, 7b), about which the lancet supply strip (5) is guided, and performing a movement in the puncturing direction during a puncturing action.

14. A method for separating a lancet tip (6a) from a lancet supply strip (5) by bending the lancet supply strip (5) in a lancing device in lengthwise direction so that the lancet tip (6a) will be lifted off the lancet supply strip (5).

15. The method as defined in Claim 14, **characterized in that** a bending mechanism (10) arranged in a lancing device is used for bending the strip (5).

## Revendications

1. Système de piqûre destiné à obtenir un échantillon de liquide corporel comprenant une bande de réserve de lancettes (5) comprenant plusieurs lancettes (6), qui sont disposées transversalement à la direction longitudinale de la bande de réserve de lancettes (5),
un système de transport pour amener les lancettes (6) de la bande de réserve de lancettes (5) de façon successive dans une position de piqûre par le transport d'une bande de réserve de lancettes (5) insérée dans le casier de réception, et
un entraînement de piqûre (8) pour amener une lancette (6) se trouvant dans la position de piqûre dans un mouvement de piqûre,
**caractérisé par**
un dispositif de flexion (10) pour fléchir une partie de la bande de réserve de lancettes (5) dans la direction longitudinale de la bande de réserve de lancettes (5) et séparer ainsi une pointe de lancette (6a) d'une lancette (6) de la bande de réserve de lancettes (5) pour une piqûre.

2. Système de piqûre selon la revendication 1, **caractérisé par**
une cassette à bande comprenant une bande de réserve de lancettes (5) avec plusieurs lancettes (6), qui sont disposées transversalement à la direction longitudinale de la bande de réserve de lancettes (5), et
un appareil de piqûre comprenant
un casier de réception pour la réception d'une cassette à bande,
un dispositif de transport pour amener les lancettes (6) de la bande de réserve de lancettes (5) de façon successive dans une position de piqûre par le transport de la bande de réserve de lancettes (5) d'une cassette à bande insérée dans le casier de réception, et
un entraînement de piqûre (8) pour amener une lancette (6) se trouvant dans la position de piqûre dans un mouvement de piqûre.

3. Système de piqûre selon l'une quelconque des revendications précédentes, **caractérisé en ce que** les pointes de lancette (6a) des lancettes (6) de la bande de réserve de lancettes (5) sont entourées d'une protection stérile, qui, lors de la flexion d'une partie contenant une lancette (6) de la bande de réserve de lancettes (5), est traversée par une pointe de lancette (6a).

4. Cassette à bande comprenant
une bande de réserve de lancettes (5) avec plusieurs lancettes (6), qui sont disposées transversalement à la direction longitudinale de la bande de réserve de lancettes (5), **caractérisée par** un dispositif de flexion (10) pour fléchir une partie de la bande de réserve de lancettes (5) dans la direction longitudinale de la bande de réserve de lancettes (5) et séparer ainsi une pointe de lancette (6a) d'une lancette (6) de la bande de réserve de lancettes (5) pour une piqûre.

5. Appareil de piqûre pour obtenir un échantillon de liquide corporel comprenant
un casier de réception pour la réception d'une bande de réserve de lancettes (5) avec plusieurs lancettes (6), qui sont disposées transversalement à la direction longitudinale de la bande de réserve de lancettes (5) et présentent une pointe de lancette (6a) pour générer une plaie de piqûre,
un système de transport pour amener les lancettes (6) de la bande de réserve de lancettes (5) de façon successive dans une position de piqûre par le transport d'une bande de réserve de lancettes (5) insérée dans le casier de réception, et
un entraînement de piqûre (8) pour mettre une lancette (6) se trouvant dans la position de piqûre dans un mouvement de piqûre,
**caractérisé par**
un dispositif de flexion (10) pour fléchir une partie de la bande de réserve de lancettes (5) dans la direction longitudinale de la bande de réserve de lancettes (5) et séparer ainsi une pointe de lancette (6a) d'une lancette (6) de la bande de réserve de lancettes (5) pour une piqûre.

6. Appareil de piqûre selon la revendication 5, **caractérisé en ce que** le dispositif de flexion (10) agit sur un bord longitudinal de la bande de réserve de lancettes (5) pour fléchir la bande de réserve de lancettes (5).

7. Appareil de piqûre selon la revendication 6, **caractérisé en ce que** le dispositif de flexion (10) agit sur un bord longitudinal de la bande de réserve de lancettes (5) pour fléchir la bande de réserve de lancettes (5) avec au moins une surface de flexion (10a) s'étendant obliquement par rapport à la direction de piqûre.

8. Appareil de piqûre selon l'une quelconque des revendications 5 à 7, **caractérisé en ce que** la partie à fléchir de la bande de réserve de lancettes (5) est déplacée pour la flexion dans le sens de piqûre.

9. Appareil de piqûre selon l'une quelconque des revendications 5 à 8, **caractérisé en ce que** l'entraînement de piqûre (8) déplace pour une piqûre une partie de la bande de réserve de lancettes (5) conjointement avec une lancette (6) dans le sens de piqûre.

10. Appareil de piqûre selon l'une quelconque des revendications 5 à 9, **caractérisé en ce que** la bande de réserve de lancettes (5) est disposée de façon échangeable dans le casier de réception.

11. Appareil de piqûre selon l'une quelconque des revendications 5 à 10, **caractérisé par** une cassette à bande disposée de façon échangeable dans le casier de réception, laquelle contient la bande de réserve de lancettes (5).

12. Appareil de piqûre selon la revendication 11, **caractérisé en ce que** le dispositif de flexion (10) est placé sur la cassette à bande.

13. Appareil de piqûre selon l'une quelconque des revendications 5 à 12, **caractérisé en ce que** le dispositif de flexion est formé par le fait qu'une lancette (6) est maintenue dans la position de piqûre conjointement avec une partie de la bande de réserve de lancettes (5) qui la supporte dans une fixation (8) de l'entraînement de piqûre, qui est disposée entre deux éléments de déviation (7a, 7b), devant lesquels la bande de réserve de lancettes (5) est guidée, de façon décalée transversalement au sens de piqûre et exécute un déplacement dans le sens de piqûre lors d'une piqûre.

14. Procédé pour séparer une pointe de lancette (6a) d'une bande de réserve de lancettes (5), par le fait que la bande de réserve de lancettes (5) est fléchie dans un appareil de piqûre dans le sens longitudinal, de sorte que la pointe de lancette (6a) se soulève de la bande de réserve de lancettes (5).

15. Procédé selon la revendication 14, **caractérisé en ce qu'**un dispositif de flexion (10) disposé dans un appareil de piqûre est utilisé pour la flexion de la bande (5).
